# EUROPEAN PATENT APPLICATION

(11) **EP 3 988 646 A1**
(43) Date of publication of application: **27.04.2022**
(21) Application number: 21209137.5
(22) Date of filing: 04.01.2017
(51) Int. Cl.: C12N 5/071, G01N 33/569, G01N 33/543

(54) **METHODS FOR IMPROVEMENT OF SEMEN QUALITY**

(30) Priority: 04.01.2016 US 201662274466 P
(62) Divisional of application: 17736220.9
(71) Applicant: University of Tennessee Research Foundation, Knoxville, TN 37996 (US)
(72) Inventor: EDWARDS, J. Lannett, Sevierville, TN 37876 (US); RISPOLL, Louisa, A, Maynardville, TN 37807 (US); SCHRICK, F. Neal, Sevierville, TN 37876 (US)
(74) Representative: Symbiosis IP Limited

(57) **Abstract**

A method for improving quality of a mammalian semen sample includes providing an insoluble carrier carrying at least one ligand that binds to an indicator of impaired sperm membrane integrity and/or motility, or the insoluble carrier carrying at least one molecule capable of binding to the at least one ligand, and contacting the semen sample and the insoluble carrier to provide a bound sperm cell population and an unbound sperm cell population, The insoluble carrier may be a plurality of beads each carrying the at least one ligand or the at least one molecule. The indicator may be associated with one or both of impaired sperm acrosome and impaired sperm plasma membrane integrity. The ligand may be an antibody. The molecule may be one of streptavidin, protein A, and an anti-ligand antibody. Kits for performing the method are provided.

## Description

This utility patent application claims the benefit of priority in U.S. provisional patent application S.N. 62/274,466 filed on January 4, 2016, the entire disclosure of which is incorporated herein by reference.

### TECHNICAL FIELD

This disclosure relates to methods for improving semen quality by removal of unhealthy sperm from semen samples.

### BACKGROUND

Use of artificial insemination in cattle has allowed the US beef and dairy industries to obtain multiple progeny from genetically superior sires from around the world. However, some bulls with high genetic merit fail to produce pregnant cows after artificial insemination. The fertility of their semen depends on the number of healthy sperm contained within; fertility decreases with increased numbers of sperm with poor motility and/or compromised membranes (Pace et al. 1981; Januskauskas et al. 2003; Dogan et al. 2013). This could be due to a failure of the Sertoli cells to remove degenerate sperm during spermatogenesis before ejaculation or to an increased susceptibility of the ejaculated semen to cryopreservation which results in reduced motility and/or membrane integrity in sperm after thawing. Consequently, this leads to lower conception and calving rates, and subsequently decreased pounds at weaning and reduced milk production.

To solve this and other problems, the present disclosure describes methods for improving quality of semen by removing unhealthy sperm before or after cryopreservation of semen, thus selectively enriching for healthy sperm. By the disclosed methods, numbers of healthy sperm are increased, semen fertility is increased, and reproductive outcome (e.g. *in vitro* embryo production, conception, pregnancy, birth of live calves, etc.) following procedures such as artificial insemination, *in vitro* fertilization, etc. is improved. As another non-limiting example, it is known that mammalian semen sexed by sorting exhibits reduced quality in comparison to unsorted semen. Accordingly, the disclosed methods find utility in improving quality of semen sexed by any suitable method, such as those disclosed in co-pending U.S. Published Patent Appl. No. 2015/0233920 and PCT patent application no. PCT/US15/16589, the entirety of the disclosures of each of which are incorporated by reference as if fully set forth herein, both entitled "Antibody for skewing sex ratio and methods of use thereof' and both filed on February 14, 2015.

### SUMMARY

In accordance with the purposes and benefits described herein, in one aspect methods are provided for improving quality of semen by removing unhealthy sperm using an insoluble carrier carrying at least one ligand that binds to an indicator of impaired sperm membrane integrity or at least one molecule capable of binding to the at least one ligand. The indicator may be associated with one or both of impaired sperm acrosome integrity and impaired sperm plasma membrane integrity. In embodiments, the molecule is selected from the group consisting of streptavidin, protein A, and an antibody. In embodiments, the indicator may be biotin and the ligand may be an antibody. In embodiments, the antibody is an anti-rabbit IgG or a binding fragment thereof. The antibody may be a polyclonal or a monoclonal antibody.

The methods include further steps of bringing the semen sample and the insoluble carrier into contact under conditions favorable for binding of the ligand to sperm with impaired motility and/or membrane integrity to provide an insoluble carrier-bound sperm cell population and an unbound sperm cell population. The methods further include a step of separating the bound sperm cell population from the unbound sperm cell population. In embodiments, the insoluble carrier comprises a plurality of beads coated with the at least one ligand or the at least one molecule and the method includes a step of admixing the semen sample and the plurality of coated beads. In embodiments, the method includes a step of separating the bead-bound sperm cell population by size exclusion filtering. In other embodiments, the method includes a step of providing an insoluble carrier that is a magnetic bead and a step of separating the bead-bound sperm cell population by magnetic separation.

In another aspect, the present disclosure provides a kit comprising reagents and optionally glassware and/or plasticware and instructions for accomplishing the above-described methods.

In the following description, there are shown and described embodiments of the disclosed methods for improving semen quality. As it should be realized, the methods are capable of other, different embodiments and their several details are capable of modification in various, obvious aspects all without departing from the methods as set forth and described in the following claims. Accordingly, the drawings and descriptions should be regarded as illustrative in nature and not as restrictive.

### BRIEF DESCRIPTION OF THE DRAWINGS

The accompanying drawing figures incorporated herein and forming a part of the specification, illustrate several aspects of the disclosed methods for methods for improving semen quality, and together with the description serve to explain certain principles thereof. In the drawings:
Figure 1 shows percentage of motile sperm after incubating with various coated insoluble carriers; and
Figure 2 shows percentage of sperm with intact acrosomal and plasma membranes after incubating with various coated insoluble carriers.

Reference will now be made in detail to embodiments of the disclosed methods improving semen quality, examples of which are illustrated in the accompanying drawing figures.

### DETAILED DESCRIPTION

Example 1. *Improving frozen-thawed bull semen with coated beads after removal of extender.* Different types of coated beads (Table 1) were individually incubated with bull sperm. Prior to incubation with coated beads, frozen-thawed semen from two bulls (Holstein) was centrifuged after dilution in HEPES-TALP/Citrate buffer (pre-warmed to 25-30°C; 114 mM NaCl, 3.2 mM KCl, 0.3 mM NaH₂PO₄, 10 mM Lactic Acid, 2 mM CaCl₂, 0.5 mM MgCl₂, 10 mM HEPES. 2 mM NaHCO₃, 0.2 mM Na Pyruvate, 0.5% BSA, 0.6% sodium citrate) to remove extender. The resulting sperm pellet was re-suspended to a concentration of 20 x 10^6 cells/mL using HEPES-TALP/Citrate buffer. The sperm suspension was transferred in 0.5 mL volumes to 2 mL round bottom tubes. Coated beads (volume indicated in Table 1) were individually added to each tube of sperm with HEPES-TALP/Citrated buffer to achieve a final volume of 1 mL. In instances where bead storage buffer was recommended to be removed by manufacturer prior to adding to cells (indicated in Table 1), beads were washed in HEPES-TALP/Citrate buffer following manufacturer recommendations. Control sample (no beads) consisted of sperm with HEPES-TALP/Citrate buffer only. Tubes were gently mixed for 30 minutes at room temperature.

**Table 1. Description of the coated beads evaluated for removal of unhealthy sperm**

| Bead Description | Manufacturer | Coating | Volume of bead (µL) | Washed |
|---|---|---|---|---|
| S-pluribead^{®} | | Polyclonal Anti-rabbit | | |
| | pluriSelect USA, San Diego, CA, USA | IgG (heavy and light chains) | 100 | No |
| M-pluribead^{®} | pluriSelect USA, San Diego, CA, USA | Polyclonal Anti-rabbit IgG (heavy and light chains) | 100 | No |
| FlowComp^{™} bead | Thermo Fisher Scientific Inc, Waltham, MA USA | Streptavidin | 20 | Yes |
| Dynabead^{®} | Thermo Fisher Scientific Inc, Waltham, MA USA | Protein A | 10 | Yes |
| BioMag^{®} particle | | Polyclonal Anti-rabbit | | |
| | Bangs Laboratories Inc, Fishers, IN, USA | IgG (heavy and light chains) | 150 | Yes |
| MACS^{®} MicroBead | Miltenyi Biotec Inc, San Diego, CA, USA | F(ab')₂ fragments of Polyclonal Anti-rabbit IgG (heavy and light chains) | 80 | No |

The unbound cells were separated from beads and bead-bound sperm using the appropriate device. For S- and M-pluribeads^{®}, 30 µm and 60 µm size exclusion sieves (pluriSelect^{®}), respectively, were employed for the non-magnetic separation; non-bound cells were passed through the mesh of sieve and collected below in a new tube whereas beads and bead-bound sperm were retained on the mesh. For FlowComp^{™} beads, Dynabeads^{®} and BioMag^{®} particles, a 6 tube magnetic separation rack was used to sequester the beads and bead-bound sperm against the side of the tube allowing the non-bound cells suspended in supernatant to be transferred to a new tube. For the MACS^{®} MicroBeads, a magnetic MS column (Miltenyi Biotec) on a MiniMACS^{™} separator (Miltenyi Biotec) attached to a MACS^{®} multistand (Miltenyi Biotec) was employed for the magnetic separation; beads and bead-bound sperm were retained within the magnetic field of the column and the non-bound cells flowed through the column to be collected in a new tube.

To assess the health of the non-bound sperm compared to control sperm, motility and membrane status were evaluated. A computer assisted sperm analyzer (CASA, Hamilton Thorne, Boston, MA, USA) was used to determine the motility of sperm. To assess the status of acrosomal and plasma membranes on sperm, a portion of the non-bound cells were simultaneously stained with SYBR-14 (Molecular Probes, division of Thermo Fisher Scientific) to distinguish sperm from non-sperm objects within the sample, phycoerythrin-conjugated peanut agglutinin (PE-PNA; GeneTex Inc, Irvine, CA, USA) to label sperm with non-intact acrosomes and DRAQ 7 (Abcam^{®}, Cambridge, MA, USA) to label sperm with compromised plasma membranes, using a modification of the procedure described in Nagy et al (2004). Specifically, sperm diluted in Dulbecco's Phosphate Buffered Saline (DPBS; Gibco^{®} Life Technologies, division of Thermo Fisher Scientific) were stained with 10 nM SYBR-14, 2.5 µg/mL PE-PNA and 3 µM DRAQ 7 for 10 minutes at 35° C then were evaluated on a BD Accuri C6 flow cytometer (BD Biosciences, San Jose, CA, USA). A minimum of 50,000 cells were evaluated for each sample.

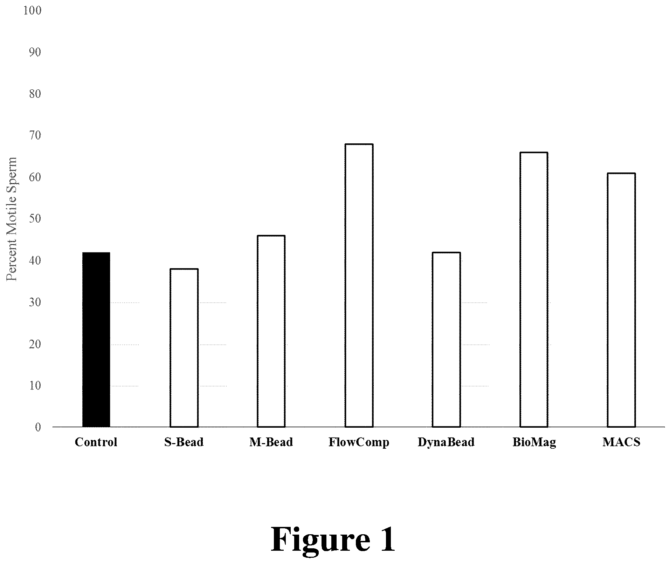

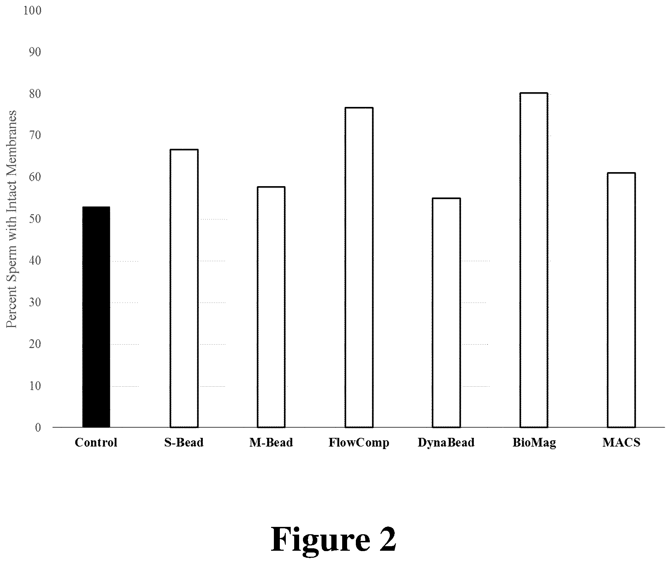

Results from the bead testing suggested that enrichment for healthy sperm (based on motility and membrane status) was feasible depending on the bead type used. With reference to Figure 1, a comparison of motility of non-bound sperm after treatment with beads to control sperm (not exposed to any bead) indicated that the percentage of motile sperm increased by ~20% or more in samples exposed to either the FlowComp^{™} beads, BioMag^{®} particles, or MACS^{®} MicroBeads whereas co-incubating sperm with S-pluribeads^{®}, M-pluribeads^{®} or Dynabeads^{®} had little to no effect on motility in the non-bound sperm population. Results from evaluation of the membrane status on the non-bound sperm (see Figure 2) showed that after exposing sperm to either S-pluribeads^{®}, FlowComp^{™} beads, BioMag^{®} particles or the MACS^{®} MicroBeads, the percent of sperm with intact acrosome and plasma membranes was increased by ~14% or higher whereas M-pluribeads^{®} or Dynabeads^{®} had little to no effect.

Example 2. *Improving frozen-thawed semen using MACS*^{®} *MicroBeads in presence of extender.* To further test the efficacy of removing unhealthy sperm using the MACS^{®} MicroBeads, two types of coatings were tested, annexin V or anti-rabbit IgG, on sperm from 4 different bulls. Prior to incubation with MACS^{®} MicroBeads, frozen-thawed semen from an individual bulls (Holstein or Senepol) was mixed with an equal volume of HEPES-TALP/Citrate. Sperm suspension was transferred in 1 mL volumes to 2 mL round bottom tubes. MACS^{®} MicroBeads were added to each tube of sperm in four different combinations: 1) none, 2) 100 µL annexin V-coated beads, 3) 100 µL anti-rabbit IgG-coated beads and 4) 100 µL annexin V-coated and 100 µL anti-rabbit IgG-coated beads. Buffer was added to samples to ensure final volume was similar between treatments. Tubes were gently mixed for 30 minutes at room temperature.

The sperm-bead suspensions were loaded onto a magnetic MS column (Miltenyi Biotec) on a MiniMACS^{™} separator (Miltenyi Biotec) attached to a MACS^{®} multistand (Miltenyi Biotec). The non-bound cells flowed through the column and were collected in a new tube. The column was removed from the separator releasing the beads and bead-bound sperm from the magnetic field. The column was washed out with 1.2 mL of HEPES-TALP buffer to recover bead-bound sperm. To assess the health of sperm after treatment and separation into non-bound and bound fractions, motility and plasma membrane status were evaluated in sperm cells stained with VIADENT (Hamilton Thorne; labels sperm with compromised plasma membranes) using the CASA instrument described above. The experiment was repeated 4 different times using a different bull each time. Data were analyzed as a randomized block design using binomial distribution and generalized linear mixed models (PROC GLIMMIX; SAS 9.4, SAS Inst., Inc, Cary, NC, USA). Treatment differences were determined using protected least significant differences and reported as least squares means ± SEM using the inverse link option.

Results from the four replicates (each on a different bull) of MACS^{®} MicroBead testing indicated that efficacy of removing sperm with poor motility or compromised plasma membranes depended on the coating on the bead employed. The non-bound fraction of sperm after exposure to anti-rabbit IgG coated beads had more motile sperm (*P* < 0.0001) with intact plasma membranes (*P* < 0.0001) compared to controls (Table 2) whereas co-incubating sperm with annexin V-coated beads did not improve the motility or plasma membrane status in the non-bound fraction compared to controls. Combining the two types of coated beads did not provide a synergistic effect on enriching for healthy sperm in the non-bound fraction (motility and membrane status results: annexin V ≤ control < anti-rabbit IgG = both; see Table 2. The differences between bead types were reflected in the degree of removal of sperm with poor motility or compromised plasma membranes through binding to the beads (bound fraction; see Table 2). The sperm which bound to beads had lower motility compared to either the unbound fractions or the control with the effect being more evident in sperm exposed to anti-rabbit IgG coated beads (controls > annexin V > anti-rabbit IgG; see Table 2). In regards to membrane status, only the bound fraction of sperm exposed to anti-rabbit IgG coated beads (with or without annexin V-coated beads) had fewer sperm with intact plasma membranes compare to controls (Table 2).

**Table 2. Results of sperm motility and plasma membrane status in non-separated and MACS^{®} MicroBead separated fractions of non-bound and bead-bound sperm**

| MACS^{®} MicroBeads | Motility (% Motile)^{∗} | | Plasma Membrane Status (% Intact)^{∗} | | | | | |
|---|---|---|---|---|---|---|---|---|
| | Non-bound | Bound | Non-bound | Bound | | | | |
| None | 34^{b} ± 7 | | 74^{b} ± 3 | | | | | |
| Annexin V | | 31^{b} ± 7 | | 17^{c} ± 6 | | 71^{c} ± 3 | | 70^{bc} ± 6 |
| Anti-Rabbit IgG | | 42^{a} ± 7 | | 6^{d} ± 2 | | 92^{a} ± 1 | | 20^{d} ± 3 |
| Both | | 41^{a} ± 7 | | 7^{d} ± 2 | | 93^{a} ± 1 | | 19^{d} ± 3 |
| | | | | | | | | |
| | *P* value^{∗∗} | | < 0.0001 | | | | < 0.0001 | |
| ^{∗}Results expressed as least squares means ± S.E.M. | | | | | | | | |
| ^{∗∗}Means with different superscript letters denote statistical difference | | | | | | | | |

Example 3. *Improving frozen-thawed semen using BioMag*^{®} *particles in presence of extender.* Prior to incubation with BioMag^{®} particles, frozen-thawed semen from individual bulls (Holstein or Senepol) was mixed with an equal volume of HEPES-TALP/Citrate. Sperm suspension was transferred in 1 mL volumes to 2 mL round bottom tubes. BioMag^{®} particles (150 µL) coated with anti-rabbit IgG were washed in HEPES-TALP/Citrate buffer prior to adding to sperm. Buffer was added to the control sample to ensure that the final volume was similar between two treatments. After gently mixing for 30 minutes at room temperature, tubes were placed onto a 6 tube magnetic separation rack for 7 minutes. The non-bound fraction suspended in supernatant was transferred to a new tube. Then HEPES-TALP/Citrate buffer was added to tubes and tubes were removed from the rack. The beads and bead-bound sperm (bound fraction) were gently pipetted to produce a homogenous suspension. To assess the health of sperm after treatment and separation into non-bound and bound fractions, motility and plasma membrane status were evaluated in sperm cells stained with VIADENT (Hamilton Thorne) using the CASA instrument. The experiment was repeated 4 different times using a different bull each time. Data were analyzed as a randomized block design using binomial distribution and generalized linear mixed models (PROC GLIMMIX; SAS 9.4, SAS Inst., Inc, Cary, NC, USA). Treatment differences were determined using protected least significant differences and reported as least squares means ± SEM using the inverse link option.

Results from the four replicates (each on a different bull) of BioMag^{®} particles testing indicated that removing sperm with poor motility may not significantly improve the quality of the non-bound fraction. The non-bound fraction of sperm after exposure to anti-rabbit IgG beads had similar motility and degree of intact plasma membranes compared to controls (see Table 3). Although sperm which bound to beads had lower motility than unbound fractions and control (P < 0.0001), no differences in percentage of sperm with intact plasma membranes were observed between groups (*P* = 0.4563; Table 3).

**Table 3. Results of sperm motility and plasma membrane status in non-separated and BioMag^{®} particle separated fractions of non-bound and bead-bound sperm**

| BioMag^{®} Particles | | Motility (% Motile)^{∗} | | | Plasma Membrane Status (% Intact)^{∗} | | | |
|---|---|---|---|---|---|---|---|---|
| | | Non-bound | | Bound | | Non-bound | | Bound |
| None | | 39^{a} ± 1 | | | | 73 ± 4 | | |
| Anti-Rabbit IgG | | 42^{a} ± 1 | | 10^{b} ± 1 | | 79 ± 3 | | 72 ± 8 |
| | | | | | | | | |
| | *P* value^{∗∗} | | < 0.0001 | | | | 0.4563 | |
| ^{∗}Results expressed as least squares means ± S.E.M. | | | | | | | | |
| ^{∗∗}Means with different superscript letters denote statistical difference | | | | | | | | |

Unless defined otherwise, all technical and scientific terms used herein have the same meaning as commonly understood by one of ordinary skill in the art to which the presently-disclosed subject matter belongs. Although any methods, devices, and materials similar or equivalent to those described herein can be used in the practice or testing of the presently-disclosed subject matter, representative methods, devices, and materials were described.

Following long-standing patent law convention, the terms "a," "an," and "the" refer to "one or more" when used in this application, including the claims. Thus, for example, reference to "a cell" includes a plurality of such cells, and so forth.

Unless otherwise indicated, all numbers expressing quantities of ingredients, properties such as reaction conditions, and so forth used in the specification and claims are to be understood as being modified in all instances by the term "about." Accordingly, unless indicated to the contrary, the numerical parameters set forth in this specification and claims are approximations that can vary depending upon the desired properties sought to be obtained by the presently-disclosed subject matter.

As used herein, the term "about," when referring to a value or to an amount of a composition, dose, sequence identity *(e.g*., when comparing two or more nucleotide or amino acid sequences), mass, weight, temperature, time, volume, concentration, percentage, *etc.,* is meant to encompass variations of in some embodiments ±20%, in some embodiments ±10%, in some embodiments ±5%, in some embodiments ±1 %, in some embodiments ±0.5%, and in some embodiments ±0.1% from the specified amount, as such variations are appropriate to perform the disclosed methods or employ the disclosed compositions.

The term "comprising", which is synonymous with "including," "containing," or "characterized by," is inclusive or open-ended and does not exclude additional un-recited elements or method steps. "Comprising" is a term of art used in claim language which means that the named elements are essential, but other elements can be added and still form a construct within the scope of the claim.

As used herein, the phrase "consisting of" excludes any element, step, or ingredient not specified in the claim. When the phrase "consists of appears in a clause of the body of a claim, rather than immediately following the preamble, it limits only the element set forth in that clause; other elements are not excluded from the claim as a whole.

As used herein, the phrase "consisting essentially of' limits the scope of a claim to the specified materials or steps, plus those that do not materially affect the basic and novel characteristic(s) of the claimed subject matter. With respect to the terms "comprising", "consisting of", and "consisting essentially of", where one of these three terms is used herein, the presently disclosed and claimed subject matter can include the use of either of the other two terms.

As used herein, the term "and/or" when used in the context of a listing of entities, refers to the entities being present singly or in combination. Thus, for example, the phrase "A, S, C, and/or O" includes A, S, C, and O individually, but also includes any and all combinations and sub-combinations of A, S, C, and O.

The term "antibody" (Ab) as used herein includes monoclonal antibodies, polyclonal antibodies, multi-specific antibodies and antibody fragments, as long as they exhibit the desired biological activity and binding characteristics. The term "polyclonal antibody" as used herein refers to an antibody obtained from a population of heterogeneous antibodies, *i.e.,* they are secreted by different B cell lineages within the body. The term "monoclonal antibody" as used herein refers to an antibody obtained from a population of substantially homogeneous antibodies, *i.e.,* the individual antibodies that make up the population are identical except for possible naturally occurring mutations. Monoclonal antibodies are highly specific, being directed against a single antigenic site.

The term "antibody" (Ab) as used herein also includes antibody fragments. An "antibody fragment" is a portion of an intact antibody, preferably the antigen binding or variable region of the intact antibody. Examples of antibody fragments include but are not limited to: Fab, Fab', F(ab')2, and Fv fragments; diabodies; linear antibodies; single-chain antibody molecules; and multispecific antibodies formed from antibody fragments.

The term "semen" as used herein refers to the fluid which contains sperm cells in mammals. Semen as used herein includes neat and diluted semen. The method finds utility in improving quality of fresh, frozen (cryopreserved), and cooled semen.

The foregoing description of a preferred embodiment of the invention has been presented for purposes of illustration and description. It is not intended to be exhaustive or to limit the invention to the precise form disclosed. Obvious modifications or variations are possible in light of the above teachings. The embodiment was chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally and equitably entitled. All references cited herein are hereby incorporated by reference in their entirety.

### References cited.

Dogan, S., et al. (2013). J Reprod Dev 59: 18-26.
Januskauskas, A., et al. (2003). Theriogenology 60: 743-58.
Nagy, S., et al. (2004). Anim Reprod Sci 80: 225-35.
Pace, M. M., etal. (1981). J Anim Sci 53: 693-701.

## Claims

1. A method for improving quality of a mammalian semen sample, comprising:
Providing (i) an insoluble carrier carrying at least one ligand, or (ii) at least one ligand and an insoluble carrier carrying at least one molecule capable of binding to the at least one ligand, wherein said ligand is an anti-rabbit igG or a binding fragment thereof;
contacting the semen sample and said insoluble carrier carrying at least one ligand or said at least one ligand and an insoluble carrier carrying at least one molecule capable of binding to the at least one ligand to provide a bound sperm cell population and an unbound sperm cell population; and
separating the bound sperm cell population from the unbound sperm cell population.

2. The method of claim 1, wherein the insoluble carrier comprises a plurality of beads each carrying the at least one ligand or the at least one molecule.

3. The method of claim 2, wherein the semen sample and the insoluble carrier are contacted by admixing.

4. The method of claim 3, including a step of separating the bound sperm cell population by size exclusion filtration.

5. The method of claim 3, wherein the plurality of beads are magnetic beads, optionally selected from group consisting of BioMag^{®} particles, MACS^{®} MicroBeads and FlowComp^{™} beads, and wherein the method includes a step of separating the bound sperm cell population by magnetic separation.

6. The method of claim 1, wherein said ligand binds to sperm cells having one or both of impaired sperm acrosome and impaired sperm plasma membrane integrity.

7. The method of claim 1, including a step of removing a seminal plasma and/or an extender, if present, before the steps of providing, mixing, and separating.

8. The method of claim 1, including selecting the mammal from the group consisting of a human, an equine animal, a bovine animal, an ovine animal, and a porcine animal.

9. The method of claim 1, including providing the semen sample as one or more of a freshly collected semen sample, a cooled semen sample, a frozen-thawed semen sample, a neat semen sample, and a diluted semen sample.

10. A kit containing reagents for improving quality of a mammalian semen sample, comprising:
a binding reagent selected from one of:
i) an insoluble carrier carrying at least one ligand; or
ii) the at least one ligand and an insoluble carrier carrying at least one molecule capable of binding to the at least one ligand,
wherein said ligand is an anti-rabbit IgG or a binding fragment thereof; and
optionally, other reagents, glass and/or plasticware, and instructions for the use of the insoluble carrier carrying the at least one ligand, or the at least one ligand and an insoluble carrier carrying at least one molecule capable of binding to the at least one ligand, to bind to and separate sperm having impaired membrane integrity and/or impaired motility.

11. The kit of claim 10, wherein the insoluble carrier comprises a plurality of beads each carrying the at least one ligand or the at least one molecule.

12. The kit of claim 11, further including a size exclusion filter.

13. The kit of claim 11, wherein the plurality of beads are magnetic beads, optionally selected from group consisting of BioMag^{®} particles, MACS^{®} MicroBeads and FlowComp^{™} beads.

14. The kit of claim 13, further including a magnetic separator.

15. The method of claim 1 or the kit of claim 10, wherein the molecule is selected from the group consisting of streptavidin, protein A, and an anti-ligand antibody.
